# EUROPEAN PATENT SPECIFICATION

(11) **EP 1 652 526 B1**
(45) Date of publication and mention of the grant of the patent: **14.01.2009**
(21) Application number: 06101005.4
(22) Date of filing: 21.10.2002
(51) Int. Cl.: A61K 31/58, A61P 25/24

(54) **Use of (11beta, 17beta)-11-(1,3-benzodioxol-5-yl)-17-hydroxy-17-(1-propynyl)-estra-4,9-dien-3-one in the treatment of major depressive disorder**
Verwendung von (11beta, 17beta)-11-(1,3-Benzodioxol-5-yl)-17-Hydroxy-17-(1-Propinyl)-estra-4,9-dien-3-on zur Behandlung von schweren Depressionen
Utilisation de (11béta, 17béta)-11-(1,3-benzodioxol-5-yl)-17-hydroxy-17-(1-propinyl)-estra-4,9-dien-3-one dans le traitement de trouble dépressif majeur

(30) Priority: 26.10.2001 EP 01204072
(43) Date of publication of application: 03.05.2006
(62) Divisional of application: 02781273.4
(73) Proprietor: N.V. Organon, 5349 AB Oss (NL)
(72) Inventor: Sennef, Cornelis, 5340 BH, Oss (NL); Peeters, B.W.M.M., Bernardus, 5340 BH, Oss (NL)
(74) Representative: Kraak, Hajo

(56) References cited:
- EP-A- 0 763 541
- WO-A-99/17779
- WO-A-02/076390
- WO-A-02/096433
- US-A- 4 814 333
- MURPHY B E ET AL: "Possible use of glucocorticoid receptor antagonists in the treatment of major depression: Preliminary results using RU 486" JOURNAL OF PSYCHIATRY AND NEUROSCIENCE, OTTAWA, CA, vol. 18, no. 5, 1 November 1993 (1993-11-01), pages 209-213, XP002091170 ISSN: 1180-4882
- AGARWAL M K, HAINQUE B, MOUSTAID N, LAZER G: "Glucocorticoid antagonists" FEBS LETTERS, vol. 217, no. 2, June 1987 (1987-06), pages 221-226, XP002195329

## Description

The present invention relates to the use of (11β,17β)-11-(1,3-benzodioxol-5-yl)-17-hydroxy-17-(1-propynyl)estra-4,9-dien-3-one (Org 34517) for the preparation of a medicament for the treatment of depression.

Major depressive disorder is a psychiatric disorder which has a lifetime prevalence of around 8 %. One of the most consistent findings in psychiatry is that patients with major depression present with alterations in the hypothalamic-pituitary-adrenal (HPA) axis. A significant percentage of depressed patients exhibit hypersecretion of the adrenal glucocorticosteroid cortisol, as manifested by elevated plasma and cerebrospinal fluid concentrations of cortisol and increased urinary free cortisol. In addition many depressed patients exhibit a clear inability to switch off endogenous cortisol release following exogenous challenge with the potent synthetic glucocorticoid dexamethasone (the so-called dexamethasone non-suppressors) (Gold P.W., et al., Clinical and biochemical manifestations of depression: relation to neurobiology of stress. New England J. Med. 319, 413-420, 1988). Other abnormalities of the HPA axis found in depressed patients are increased cortisol response to corticotrophin, a blunted corticotrophin response to CRH (corticotrophin releasing hormone), adrenal and pituitary enlargement and diminished glucocorticoid negative feedback (for a review see Holsboer, F. and Barden, N.: Antidepressants and Hypothalamic-Pituitary-Adrenocortical regulation. Endocrine Reviews 1996, 17, 187-205). These observations have been interpreted to suggest a causal relationship between disturbed functioning of the HPA axis and the pathology of depression (Murphy, B.E.P.: Steroids and Depression. J. of Steroid Biochem. and Mol. Biol. 1991, 38, 537-559). Therapeutic efficacy of classical antidepressants has been shown to be preceded by or to coincide with restoration of the disturbed HPA axis in depression (Holsboer and Barden, 1996, *supra).* It has been postulated that any intervention which can restore this HPA dysfunction may have antidepressant potential. One type of such intervention is the administration of glucocorticoid synthesis inhibitors, as has been shown in patients suffering from Cushing's syndrome, which is a condition in which high cortisol levels are reported as a result of adrenal gland malfunction (due to a pituitary tumour or a secondary tumour, both producing the cortisol secretagogue ACTH). The depressive symptoms associated with Cushing's disappear relatively quickly with the return of cortisol levels to normal. Such treatment may involve removal of the offending tumour or treatment with cortisol synthesis inhibitors such as metyrapone, ketoconozole, or aminoglutethimide (Murpy, B.E.P., Steroids and Depression. J. Steroid Biochem & Mol. Biol. 38, 537-558, 1991). Similarly, relatively recent clinical trials have demonstrated that cortisol synthesis inhibitors can be used to ameliorate depressive symptoms in severe, treatment-resistant non-Cushing depressives (Murphy, B.E.P., Neuroendocrine responses to inhibitors of steroid synthesis in patients with major depression resistent to antidepressant therapy. Can. J. Psych. 43, 279-286, 1998; see also US Patent 4,814,333 (Ravaris, C.L.): *Method for treatment of hypercortisolemic, depressed patients.).* Drawbacks of the use of cortisol synthesis inhibitors to lower plasma cortisol levels are their high toxicity and their relatively low degree of selectivity for inhibition of cortisol synthesis versus synthesis of other endogenously manufactured steroids (such as mineralocorticoids and sex steroids) combined with the risk for the induction of adrenal insufficiencies. A further serious disadvantage is that the onset of therapeutic effect of these cortisol synthesis inhibitors is as long as that observed with classical antidepressants (e.g., several weeks).

Another type of intervention is the use of direct glucocorticoid receptor (GR) antagonists, which have much more specific pharmacological effects as compared to synthesis inhibitors and which may help restore HPA activity. Small scale pilot clinical studies have been conducted in order to study the antidepressant activity of the non-selective glucocorticoid receptor antagonist RU 486 (mifepristone; 17β-hydroxy-11β-(4-dimethylaminophenyl)-17α-(1-propynyl)estra-4,9-dien-3-one; Murphy, B.E.P. et al . J. Psychiat. Neurosc. 18, 209-213, 1993). Relatively high doses mifepristone, in the range of 8 -12 mg/kg/day, over a relatively short period of time (4 days), was also shown to be effective in the treatment of psychosis associated with psychotic major depression (international Patent Application WO 99/17779; Schatzberg and Belanoff). More recently (Nemerott, C., Remeron Scientific Expert Meeting, Budapest, March 29-April 1, 2001) it was demonstrated in a Phase IIB continuation of this study, that both the number of responders as well as the efficacy of the psychosis treatment increased with increasing daily dose of mifepristone as measured by the change in Brief Psychiatric Rating Scale (50 mg-33% change; 600 mg-40 % change and 1200 mg-52 % change).

These data suggest that a higher dose of glucocorticoid receptor antagonist is correlated with a higher clinical efficacy.

Selective glucocorticoid receptor antagonists, which are structurally related to mifepristone, which lack appreciable affinity for mineralocorticoid, estrogen and androgen receptors, and which have low affinity for the progesterone receptor have been disclosed in European Patent 763 541 B1 (Akzo Nobel N.V.) as being potentially useful in the prevention and treatment of glucocorticoid dependent diseases or symptoms, like Cushing syndrome, diabetes, glaucoma, sleep disturbances, depression, anxiety, atherosclerosis, hypertension, adiposity, osteoporosis and withdrawal symptoms from narcotics.

The antiglucocorticoid activity of (11β,17β)-11-(1,3-benzodioxol-5-yl)-17-hydroxy-17-(1-propynyl)estra-4,9-dien-3-one, a compound specifically disclosed in EP 763 541 B1, and which will be referred to as Org 34517, was found to be dose related as measured by the procedure described by Kloosterboer et al (J. Steroid Biochem 31, 567-571, 1988): the effect (weight gain) of orally applied Org 34517 on body weight, adrenals, thymus and spleen of immature dexamethasone treated male rats proved to increase with the dose (from 10 mg/kg to 40 mg/kg).

Org 34517 was found to be less potent (52%) than mifepristone in *in vitro* binding to the glucocorticoid receptor. The threshold dose for demonstrating *in vivo* (rat) antiglucocorticoid effects was likewise much higher for Org 34517 (20 mg/kg) than for mifepristone (5 mg/kg). In agreement with the relatively lower antiglucocorticoid activity, a clinical study wherein the antiglucocorticoid activity in (healthy) humans was estimated on the basis of the cortisol increase or on the amount of dexamethason antagonism, upon administration of Org 34517, revealed Org 34517 to have a potency relative to mifepristone of 10-25% and 17%, respectively.

These data would indicate that clinical useful effects during treatment of a glucocorticoid dependent disease should be expected at daily doses of Org 34517 which would be much higher than for the more potent antiglucocorticoid mifepristone.

It has now been unexpectedly found in clinical studies that patients suffering from major depressive disorder should be treated with a daily dosage of Org 34517 which does not exceed 300 mg. Administration of such a relatively low dose of the antiglucocorticoid Org 34517 results in fast onset of antidepressant effect as compared to the onset of antidepressant effect in patients treated with a daily dosage of 450 mg of Org 34517 or more. The preferred daily dosage of Org 34517 ranges between 150 and 300 mg.

An advantage of these low doses is that potential side effects which might result from the residual antiprogestagenic activity of the compound, and which could lead to interference with the normal female menstrual cycle, are kept to the minimum.

Glucocorticoids are extremely important hormones, which play key roles in the coping mechanisms that animals (including man) have at their disposal against internal and external stressors. Pharmacologically effective dosages of glucocorticoid receptor antagonists, such as Org 34517 and RU 486, will block physiological action of endogenous glucocorticoids and may thereby induce risks when stressors affect the organism. The low dose treatment regimen of the present invention thus warrants minimal increases in susceptibility for the induction of risks.

In a preferred embodiment the invention is concerned with a medicament comprising a daily dosage of Org 34517 between 150 and 300 mg, for the treatment of patients suffering from a major depressive disorder and who have a plasma cortisol level, as measured by the afternoon cortisol test, which is higher than 10 µg/dl. Such major depressive disorder patients, having a disturbed regulation of the hypothalamus-pituitary-adrenal (HPA) axis, show a short onset of action of the antidepressant effect of Org 34517. The low dosage medicament of the invention is especially effective with regard to the onset of action of the antidepressant effect in patients classified as dexamethasone non-suppressors, i.e. patients which demonstrate non-suppression in the dexamethason suppression test.

Org 34517, (11β,17β)-11-(1,3-benzodioxol-5-yl)-17-hydroxy-17-(1-propynyl)estra-4,9-dien-3-one, can be prepared as descibed in European Patent P 763 541 B1 (Akzo Nobel N.V.).

The pharmaceutical preparations, or compositions, for use according to the invention comprise (11β,17β)-11-(1,3-benzodioxol-5-yl)-17-hydroxy-17-(1-propynyl)estra-4,9-dien-3-one in admixture with pharmaceutically acceptable auxiliaries. The term "acceptable" means being compatible with the other ingredients of the composition and not deleterious to the recipients thereof. The compositions can be prepared in accordance with standard techniques such as those described in the standard reference Gennaro A.R. et al., Remington: The Science and Practice of Pharmacy, (20th ed., Lippincott Williams & Wilkins, 2000, Part 5: Pharmaceutical Manufacturing).

Compositions include e.g. those suitable for oral, sublingual, topical or rectal administration, and the like, all in unit dosage forms for administration.

For oral administration, which is the preferred route, the active ingredient may be presented as discrete units, such as tablets, capsules, powders, granulates, solutions, and suspensions.

The invention is illustrated in the following examples:

### Example 1.

### Treatment of major depressive patients with Org 34517

A double blind, 4 week, paroxetine controlled study of Org 34517 in depressed patients was carried out. Paroxetine is a selective serotonin re-uptake inhibitor which is recognized as an effective antidepressant for major depression. Patients were selected which had a primary depressive disorder fulfilling the diagnostic criteria of a Major Depressive Disorder (MDD) as defined by the DSM-IV for recurrent (296.3) episodes, and who had a severity of depression which resulted in a total score of at least 22 on the HAMD-21 (HAMilton Rating Scale for Depression; see Hamilton, M. "A rating scale for depression." J. Neurol. Neurosurg. Psychiat. 1960, 23, 56-62) scale at baseline. Patient had an episode of depression which had lasted at least 2 weeks before baseline.

Patients were randomly allocated to one of three treatment groups. Group I patients (Org 150 group: 50 patients) received 2 capsules with 75 mg of Org 34517 and one placebo (total daily dose 150 mg) for the first 2 weeks and 2 capsules with 75 mg Org 34517 and 1 capsule with 150 mg (total daily dose 300 mg) the next 2 weeks; Group II patients (Org 450 group: 46 patients) received 3 capsules with 150 mg Org 34517( total daily dose 450 mg) in the first 2 weeks and 4 capsules of Org 34517 (total daily dose 600 mg) in the next 2 weeks; Group III patients (paroxetine group: 44 patients) received 2 capsules with 10 mg paroxetine and one placebo capsule (total daily dose 20 mg) for the first 2 weeks, followed by 2 capsules of 10 mg and one capsule of 20 mg paroxetine (total daily dose 40 mg) in the next 2 weeks. Medication was administered orally in the morning. Efficacy assessment was done on days 4, 7, 10, 14, 21, 28 and 35 by using the 21-item HAMD scale.

Afternoon cortisol plasma levels (ACT=afternoon cortisol test) were measured as described by Halbreich et al (J. Clin. Endocrinol. Metab. 54(6), 1262, 1982).

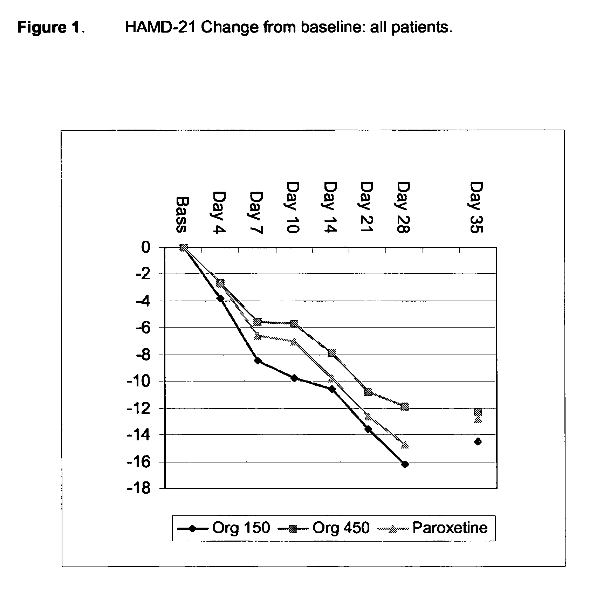

Results for all the patients are shown in Figure 1. These data demonstrate a faster onset of action of the antidepressant effect for the 150 mg treatment group as compared with paroxetine, measured as an approximate 2 point difference on the Hamilton scale on day 10.

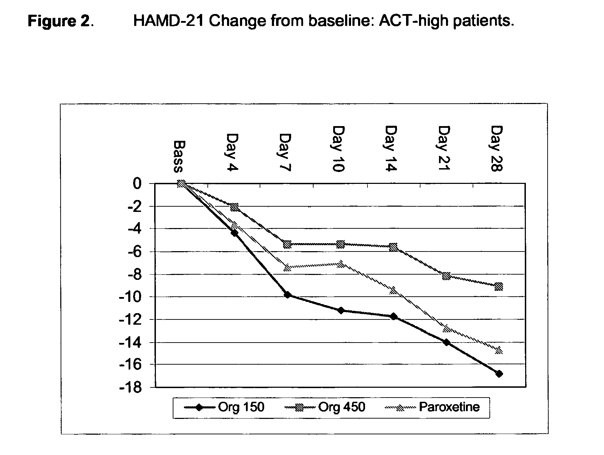

Results for patients who had an afternoon plasma cortisol level at the start of treatment higher than 10 µg/dl are shown in Figure 2. These data again demonstrate a faster onset of action of the antidepressant effect for the 150 mg treatment group as compared with paroxetine, measured as an approximate 4 point difference on the Hamilton scale on day 10.

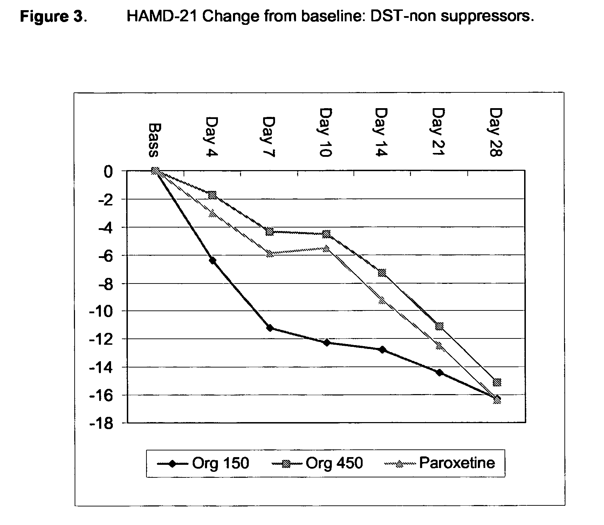

Results for the patients who were diagnosed at the start of treatment as dexamethason non-suppressors (non-suppression in the dexamethason suppression test) are shown in figure 3. These data demonstrate a pronounced difference in the onset of action of the antidepressant effect for the 150 mg treatment group as compared with the paroxetine treatment group, measured as an approximate 6 point difference on the Hamilton scale on day 10.

## Claims

1. The use of compound (11β,17β)-11-(1,3-benzodioxol-5-yl)-17-hydroxy-17-(1-propynyl)estra-4,9-dien-3-one for the preparation of a medicament for the treatment of a patient suffering from a major depressive disorder, **characterized in that** the patient is having a plasma cortisol level higher than 10 µg/dl and with the medicament the compound is to be applied in a daily dosage between 150 and 300 mg.

2. The use according to claim 1, **characterized in that** the patient is **characterized by** non-suppression of plasma cortisol level in the dexamethasone suppression test.

## Patentansprüche

1. Verwendung der Verbindung (11β, 17β)-11-(1,3-Benzodioxol-5-yl)-17-hydroxy-17-(1-propynyl)estra-4,9-dien-3-on für die Herstellung eines Medikamentes für die Behandlung eines Patienten, der an schweren depressiven Störungen leidet, **dadurch gekennzeichnet, dass** der Patient ein Plasmacortisolniveau hat, welches grösser als 10 µg/dl ist, und wobei das Medikament der Verbindung in einer täglichen Dosis zwischen 150 und 300 mg anzuwenden ist.

2. Verwendung nach Anspruch 1, **dadurch gekennzeichnet, dass** der Patient **gekennzeichnet ist durch** die Nichtsuppression des Plasmacortisolniveaus in dem Dexamethason-Hemmtest.

## Revendications

1. Utilisation du composé (11β,17β)-11-(1,3-benzodioxol-5-yl)-17-hydroxy-17-(1-propynyl)-estra-4,9-dién-3-one pour la préparation d'un médicament destiné au traitement d'un patient souffrant d'un trouble dépressif majeur, **caractérisée en ce que** le patient a un taux de cortisol plasmatique supérieur à 10µg/dl et **en ce que** par le médicament, le composé est administré à une dose quotidienne comprise dans la plage allant de 150 à 300 mg.

2. Utilisation selon la revendication 1, **caractérisée en ce que** le patient est **caractérisé par** une non-suppression du taux de cortisol plasmatique dans le test de freinage à la dexaméthasone.
